# EUROPÄISCHE PATENTSCHRIFT

(11) **EP 4 216 889 B1**
(45) Veröffentlichungstag und Bekanntmachung des Hinweises auf die Patenterteilung: **27.11.2024**
(21) Anmeldenummer: 21786345.5
(22) Anmeldetag: 24.09.2021
(51) Int. Cl.: A61F 9/008

(54) **ANORDNUNG ZUR LASERBEARBEITUNG VON AUGENTRÜBUNGEN**
ASSEMBLY FOR LASER TREATMENT OF OCULAR OPACITIES
ENSEMBLE POUR TRAITEMENT LASER D'OPACITÉS OCULAIRES

(30) Priorität: 25.09.2020 DE 102020212084
(43) Veröffentlichungstag der Anmeldung: 02.08.2023
(73) Patentinhaber: Carl Zeiss Meditec AG, 07745 Jena (DE)
(72) Erfinder: HACKER, Martin, 07745 Jena (DE); GRATZKE, Alexander, 07745 Jena (DE); MERKEL, Stephan, 07551 Gera (DE)
(74) Vertreter: Kintzel, Klaus-Peter
(86) Internationale Anmeldenummer: PCT/EP2021/076319
(87) Internationale Veröffentlichungsnummer: WO 2022/063961

(56) Entgegenhaltungen:
- DE-A1- 102011 103 181
- US-A1- 2014 257 257
- US-A1- 2018 028 354
- US-A1- 2019 105 519

## Beschreibung

Die vorliegende Erfindung betrifft eine Anordnung zur Laserbearbeitung von Augentrübungen.

Der Glaskörper besteht aus einer meist klaren, gelartigen Substanz im Augeninneren zwischen der Linse und der Netzhaut. In jungen Jahren ist der Glaskörper vollkommen durchsichtig und hat Kontakt zur Netzhaut. Im Laufe des Lebens verflüssigt sich der Glaskörper und löst sich zunehmend von der Netzhaut ab, was als hintere Glaskörperabhebung bezeichnet wird. Dies ist ein normaler Alterungsprozess, der sich üblicherweise nach dem 50. Lebensjahr abspielt. Die abgelösten Glaskörperanteile fallen im Inneren des Auges zusammen und die Gerüstsubstanzen und Verdichtungen des Glaskörpers werden für die Patienten sichtbar. Da sie sich auch über das Gesichtsfeld bewegen können werden sie auch als Floater bezeichnet. Oft liegen als Ursache von Floatern nach der Ablösung des Glaskörpers auch membranartige Strukturen an der posterioren Seite des Glaskörpers vor, z.T. sogar Blutreste, falls es bei der Glaskörperablösung zu Netzhautverletzungen kam. In selteneren Fällen können Floater auch bei Stoffwechselproblemen als kristallartige Ausfällungen im Glaskörper vorliegen.

Auch wenn Floater meistens keine krankhafte Ursache haben, sind sie nicht so harmlos wie allgemein angenommen, weil sie die Lebensqualität und auch Arbeitsproduktivität der Betroffenen teilweise erheblich beeinträchtigen können.

Besonders gegen einen hellen Hintergrund, z. B. beim Arbeiten am Computer, beim Lesen oder beim Blick gegen den blauen Himmel oder Schnee werden diese Trübungen wahrgenommen und stören das Sehvermögen. Besonders störend können Floater sein, die beim Lesen infolge der Lesebewegungen in den zentralen Sichtbereich hinein und hinaus geschleudert werden.

Weil sie oft die Form einer "fliegende Mücke" haben, werden sie - aus dem Französischen - mit dem Fachausdruck "Mouches-Volantes" beschrieben. Die Trübungen können aber auch andere Formen haben, z. B. ast-, ring- oder sternförmig sein oder auch als Punktwolken vorliegen. Im Folgenden wird für die zu behandelnden Glaskörpertrübungen, unabhängig von dessen Art bzw. Form der Begriff "Floater" verwendet.

Floater verschwinden i.A. nicht ohne Behandlung, weil das Immunsystem sie nicht als abnormal erkennt und daher nicht abbaut. Von den Betroffenen lassen sie sich kaum ignorieren oder übersehen. Bestimmte Floater-Typen, wie diejenige durch Blutreste nach Netzhautblutungen, werden z.T. vom Körper resorbiert, auch wenn dies oft Wochen oder Monate benötigt.

Bei einer sogenannten Vitrektomie wird nach Eröffnung des Auges mit Schneidinstrumenten der Glaskörper teilweise (Kernvitrektomie) oder vollständig zerkleinert, abgesaugt und entfernt. Ein derartiger Eingriff wird routinemäßig bei Netzhautablösungen oder Peeling epiretinaler Membranen durchgeführt, wird allerdings zur Beseitigung der umschriebenen Glaskörpertrübungen meist als unverhältnismäßige Therapie angesehen. Zudem ist die Vitrektomie invasiv, erfordern einen Klinikaufenthalt und birgt die mit einem chirurgischen Eingriff verbundenen Risiken, insbesondere häufig die Induzierung einer Katarakt, seltener einer Netzhautablösung und sehr selten, aber möglich einer Endophthalmitis.

Mit der sogenannten Laser-Vitreolyse bietet sich jetzt eine risikoarme Behandlungsalternative an. Die Laser-Vitreolyse ist eine schonende, risikoarme und schmerzfreie Laserbehandlung, mit der Glaskörpertrübungen vaporisiert oder zerstäubt werden können, ohne das Auge zu eröffnen.

Bei der Laser-Vitreolyse werden kurze Laserlichtpulse auf die Glaskörpertrübungen gerichtet, um dort auf Grund der hohen Laserintensität im Fokusbereich einen optischen Durchbruch bzw. eine Photodisruption zu erzielen. Die Floater und der sie umgebende Glaskörper nehmen die Laserenergie auf, es bildet sich ein schneidendes oder expandierendes Laserplasma, wodurch die Floater vaporisiert und/oder zerkleinert werden und sich dadurch auflösen können. Die Behandlung ist schmerzarm und ohne Infektionsrisiko. Mit der Laser-Vitreolyse steht ein sicheres Verfahren zur schonenden Behandlung von störenden Glaskörpertrübungen zur Verfügung, wenn sichergestellt werden kann, dass wichtige und empfindliche Augenstrukturen nicht durch den Laser geschädigt werden, wie z.B. der Kapselsack, die Kristalllinse oder Netzhautbereiche, insbesondere die Makula.

Allerdings ist der Erfolg der Behandlung von der Art der Floater abhängig. Besonders erfolgreich ist die Behandlung bei sogenannten Weiß-Ringen. Gewebestränge lassen sich durchtrennen und die Gewebsverdichtungen, die für die störenden Schatten verantwortlich sind, können beseitigt werden.

Bereits seit über 3 Jahrzehnten werden Floater mit YAG-Lasern (insbesondere als Nd:YAG bei 1064nm) behandelt (Brasse, K., Schmitz-Valckenberg, S., Jünemann, A. et al. Ophthalmologe (2019) 116: 73. https://doi.org/10.1007/s00347-018-0782-1). Aber selbst mit den bisherigen High End Geräten kann nur der vorderste Bereich des Glaskörpers mit Präzision und Zielsicherheit behandelt werden. Im tieferen Glaskörperbereich sind diese Laser nicht präzise genug. Aber gerade dort liegen die meisten Glaskörpertrübungen, da sie ja oft das Resultat posteriorer Glaskörperablösungen sind. YAG-Laser werden in der Ophthalmologie häufig zur Iridotomie bei Glaukomerkrankungen und zur Nachstarbehandlung eingesetzt, d.h. zur Entfernung von Trübungen oder gar einer Nachstarmembran auf Kunstlinsenimplantaten infolge von Zellüberwuchs. Frequenzverdoppelte YAG-Laser mit Laserstrahlung im Grünen (532nm) werden darüber hinaus auch zur Netzhautkoagulation eingesetzt, beispielsweise bei Blutungen oder Netzhautablösungen. Seltener kommen YAG-Laser auch zur Phakoemulsifikation bei einer Kataraktoperation zum Einsatz, also der Verflüssigung der eingetrübten und verhärteten natürlichen Linse. In diesem Fall aber eher als Er:YAG Laser mit 2940nm Wellenlänge und höherer Wasser- bzw. Gewebeabsorption, die dann auch oft aufwändig per endoskopischer Lasereinführung mit Spiegelführung in das Auge eingebracht werden muss.

Nach dem bekannten Stand der Technik existieren bereits zahlreiche Lösungen zur Laserchirurgie von Gewebe des Auges, insbesondere im Glaskörper.

So beschreibt die DE 10 2011 103 181 A1 eine Vorrichtung und ein Verfahren zur Femtosekunden-Laserchirurgie von Gewebe, insbesondere im Glaskörper des Auges. Die Vorrichtung besteht aus einem Ultrakurzpulslaser mit Pulslängen im Bereich von ca. 10fs-1ps, insbesondere ca. 300fs, Pulsenergien im Bereich von ca. 5µJ-5µJ, insbesondere ca. 1-2µJ und Pulsrepetitionsraten von ca. 10kHz-10 MHz, insbesondere 500 kHz. Das Lasersystem ist mit einem Scanner-System gekoppelt, welches die räumliche Variation des Fokus in drei Dimensionen ermöglicht. Die Vorrichtung besteht neben diesem therapeutischen Laser-Scanner-Optik-System weiterhin aus einem damit gekoppelten Navigationssystem.

Die US 2006/195076 A1 beschreibt System und Verfahren zur Herstellung von Einschnitten in Augengewebe in verschiedenen Tiefen. Das System und das Verfahren fokussieren Licht, möglicherweise in einem Muster, auf verschiedene Brennpunkte, die sich in verschiedenen Tiefen innerhalb des Augengewebes befinden. Mit einer segmentierten Linse können mehrere Brennpunkte gleichzeitig erstellt werden. Optimale Einschnitte können erzielt werden, indem das Licht nacheinander oder gleichzeitig in verschiedenen Tiefen fokussiert wird, eine erweiterte Plasmasäule und ein Strahl mit einer verlängerten Taille erzeugt werden. Die hier beschriebenen Techniken können unter anderem auch verwendet werden, um neue ophthalmologische Verfahren durchzuführen oder bestehende Verfahren zu verbessern, einschließlich Dissektion von Gewebe im posterioren Pol, wie beispielsweise Floater, Membranen und die Retina.

Auch die US 2014/257257 A1 beschreibt ein System und sein Verfahren zum Behandeln von Zielgewebe im Glaskörper eines Auges, umfassend eine Lasereinheit zum Erzeugen eines Laserstrahls und einen Detektor zum Erzeugen eines Bildes des Zielgewebes. Das System enthält auch einen Computer, der einen Brennfleckpfad zum Emulgieren des Zielgewebes definiert. Ein Komparator, der mit dem Computer verbunden ist, steuert dann die Lasereinheit, um den Brennpunkt des Laserstrahls zu bewegen. Diese Fokuspunktbewegung wird durchgeführt, um das Zielgewebe zu behandeln, während Abweichungen des Fokuspunkts vom definierten Fokuspunktpfad minimiert werden.

Die US 2015/342782 A1 betrifft ebenfalls ein System und ein Verfahren zur Verwendung eines computergesteuerten Lasersystems bereitgestellt, um eine partielle Vitrektomie des Glaskörpers in einem Auge durchzuführen. Operativ wird zunächst ein optischer Kanal durch den Glaskörper definiert. Glasartige und suspendierte Ablagerungen (Floater) im optischen Kanal werden dann abgetragen und in einigen Fällen aus dem optischen Kanal entfernt (z. B. abgesaugt). In einigen Fällen kann eine klare Flüssigkeit in den optischen Kanal eingeführt werden, um das abgetragene Material zu ersetzen und dadurch eine ungehinderte Transparenz im optischen Kanal herzustellen. Allgemein betrifft die vorliegende Erfindung Systeme für ophthalmologische Laseroperationen. Insbesondere betrifft die vorliegende Erfindung Systeme zum Verwenden gepulster Laserstrahlen zum Entfernen sogenannter Floater. Die US 2018/028354 A1 beschreibt ebenfalls ein Verfahren und ein System für einen ophthalmologischen Eingriff an einem Auge. Anhand eines Bildes von mindestens einem Teil des Auges werden unerwünschte Merkmale identifiziert. Als unerwünschte Merkmale in der Glaskörperhöhle gelten glasige Trübungen, die die Sicht beeinträchtigen, wie beispielsweise Floater. Nach dem Identifizieren und Lokalisieren der Floater durch ein Bildverarbeitungssystem werden diese nach Bestätigung durch einen Arzt automatisiert mit Laserpulsen "beschossen". Die Laserenergie verdampft zumindest einen Teil einer glasartigen Opazität. Dieser Vorgang wird wiederholt, bis die Trübung des Glaskörpers beseitigt ist. Der gesamte Vorgang wird für jede Trübung des Glaskörpers wiederholt, bis die Flüssigkeit des Glaskörpers als ausreichend klar erachtet wird.

Die US 2019/105519 A1 offenbart eine Ultraschall-basierte Lösung zur Augenbehandlung durch Kavitation. Dabei kann der Ultraschallstrahl mit einer Querschnittsgröße innerhalb eines Bereichs von etwa 50 µm bis etwa 200 µm an jeder von mehreren Stellen fokussiert und gepulst werden, um eine Vielzahl von Kavitationszonen an jeder der Zielregionen bereitzustellen. Die hochintensiven fokussierten Ultraschallimpulse (HIFU) können so konfiguriert werden, dass sie eine vorübergehende Kavitation erzeugen, die für die Erweichung des Gewebes hilfreich sein kann. Für chirurgischen Verfahren zur Entfernung von Gewebe kann die Intensität des Ultraschallstrahls gegebenenfalls ausreichend hoch eingestellt werden, um eine sichtbare Kavitation von Gewebe zu ermöglichen.

Eine weitere, laserbasierte Lösung zur Durchführung von ophthalmologischen Eingriffen an einem Auge zur Entfernung unerwünschter Merkmale wird in der US 2018/028354 A1 beschrieben. Ausgehend von einem Bild zumindest eines Teils des Auges werden von einer Datenverarbeitungseinheit die Sperrzone bestimmt und unerwünschte Merkmale identifiziert. Von der Datenverarbeitungseinheit wird eines der unerwünschten und zu entfernenden Merkmale außerhalb der Sperrzone ausgewählt. Nach einer Bestätigung für die Entfernung steuert eine Steuereinheit einen Laser, um die Entfernung zumindest eines Teils des unerwünschten Merkmals durchzuführen. Die beispielhaften Ausführungsformen beziehen sich insbesondere auf Mechanismen zum Entfernen unerwünschter Merkmale in der Glaskörperhöhle, beispielsweise Floater. Mit der hier offenbarten Lösung wird ein Arzt besser und leichter in der Lage sein, Glaskörpertrübungen ohne Operation zu entfernen.

Ein von der Firma ELLEX beschriebenes Verfahren (Produkt-Broschüre der Firma Ellex Medical Pty Ltd.; "Tango Reflex - Laser Floater Treatment"; PB0025B; 2018; (http://www.ellex.com)) sieht die Nutzung eines gepulsten Nanosekundenlasers (YAG) vor, um Glaskörpertrübungen zu zerlegen oder durch Umwandlung in Gas vollständig zu beseitigen. Mit einem Pilot-Laserstrahl wird das Zielgebiet (Floater) anvisiert und danach mit ein oder mehrere Therapielaserpulsen "beschossen". Dabei werden sowohl der Pilot-Laserstrahl aus auch die Therapielaserpulse vom Anwender manuell ausgelöst. Eine derartige manuelle Laser-Behandlung besteht typischerweise aus zwei, jeweils 20-60 Minuten dauernden Einzelbehandlungen.

Die noch nicht veröffentlichten Anmeldungen DE 10 2019 007 147.6 und DE 10 2019 007 148.4 beschreiben Systeme zur Laser-Vitreolyse von Floatern, die die sichere und präzise Zerstäubung von Glaskörpertrübungen (Floatern) auf Basis einer Kombination eines Bearbeitungslasers mit einem OCT- oder einem OCDR-System ermöglichen. Unter einem OCDR- System (Optical Coherence Domain-Reflectometry) wird hierbei ein System zur interferometrischen Gewinnung von eindimensionalen Streuprofilen verstanden, während unter OCT (Optical Coherence Tomography) die 2- oder 3-dimensionale Bildgebung gemeint ist. In beiden Fällen sollen auch Varianten mit Aufnahmesequenzen (d.h. Film) mit eingeschlossen sein. Dabei werden Mindestabstände zu empfindlichen Augenstrukturen sichergestellt und die Aktivierung des Lasers bevorzugt nur dann erlaubt, wenn der Fokus des Bearbeitungslasers und der zu bearbeitende Floater ausreichend genau zueinander positioniert sind.

Bei beiden Ansätzen wirkt sich nachteilig aus, dass der Arzt die ihm zur Verfügung stehende, gewohnte 2-dimensionale Frontalansicht des Auges mit den 3-dimensionalen Aufnahmen des OCT- oder OCDR-Systems unter Einsatz seines räumlichen Vorstellungsvermögens ständig kombinieren muss, was bei der zeitkritischen bzw. schnellen Interaktionen mit dem Patienten extrem schwierig ist.

Die Verwendung von Laserenergie im Rahmen einer Laser-Vitreolyse ist nicht invasiv und vermeidet die Nachteile chirurgischer Eingriffe, ist jedoch auch mit Nachteilen bzw. Risiken verbunden.

So kann das Zielen des Lasers schwierig sein. Da der Arzt den Glaskörper entlang des Strahlengangs betrachtet, kann es schwierig sein, die Tiefe der Position der Netzhaut, die Tiefe der Trübung des Glaskörpers oder andere relevante Merkmale zu bestimmen. Infolgedessen besteht die Gefahr, dass die Trübung des Glaskörpers verfehlt und/oder das Auge verletzt wird.

Insbesondere erweist sich die Behandlung positionsveränderlicher und schwer erkennbarer, weitgehend transparenter Floater, die als Phasenobjekte trotzdem störende Schatten auf der Netzhaut erzeugen können, als schwierig.

Die Anwendung von Laserenergie kann auch zu einer zusätzlichen Bewegung der Trübungen des Glaskörpers führen, was die Behandlung noch mehr erschwert. Somit kann es nötig sein, dass der Arzt den Laser nach jeder Anwendung von Laserenergie neu ausrichten muss. Dies kann viel Zeit in Anspruch nehmen. Daher ist eine Behandlung mit Laserenergie aufwändig und sowohl für den Patienten als auch für den Arzt belastend.

Ein weiteres mögliches Problem sind unvollständige Glaskörperabhebungen, die zu lokalen Vitreotraktionen bis hin zu Netzhautablösungen führen können. Eine Laserbehandlung im Glaskörper kann, durch die sich in dessen Folge ausbreitenden Schockwellen zu Veränderungen der Kräfteverhältnisse im Glaskörper führen und dadurch beispielsweise Spannungen an der Netzhaut verursachen.

Letztendlich erweist sich auch die Behandlung von solchen Floatern als besonders schwierig, die sich nahe an empfindlichen Strukturen des Auges befinden. Die Laserstrahlung kann hier zu Schädigungen von Netzhaut, Augenlinse oder Makula führen.

Der vorliegenden Erfindung liegt die Aufgabe zugrunde eine Lösung zur Laserbearbeitung von Augentrübungen zu entwickeln, die die Nachteile der bekannten technischen Lösungen behebt und eine Möglichkeit bietet 2-dimensionale Ansichten des Auges mit den 3-dimensionalen Aufnahmen des Messsystems auf einfache Art zu kombinieren und so deren Handhabung für den Bediener erleichtern. Zudem soll die Lösung leicht zu implementieren und wirtschaftlich kostengünstig sein und eine einfachere, schnellere und vor allem sicherere Behandlung störender Glaskörpertrübungen durch Laser-Vitreolyse ermöglichten.

Diese Aufgabe wird mit der Anordnung zur Laserbearbeitung von Augentrübungen bestehend aus einem Messsystem zur Gewinnung von Tiefeninformationen von Augenstrukturen, einem Lasersystem zum Zerkleinern von Augentrübungen mit optischen Elementen zur Kopplung von Mess- und Lasersystem, einer Eye-Tracker-Einheit zum Detektieren axialer Augenbewegungen, einer Anzeigeeinheit und einer Steuer- und Bedieneinheit, dadurch gelöst, dass das Messsystem ausgebildet ist Tiefeninformationen von Augenstrukturen in Form von Tiefenprofilen zur Verfügung zu stellen, dass die Anzeigeeinheit ausgebildet ist zumindest eine 2D-Abbildung des Auges als Livebild darzustellen, dass die Steuer- und Bedieneinheit ausgebildet ist aus den Tiefenprofilen die Tiefenlage von Augenstrukturen relativ zur Tiefenlage des Laserfokus zu bestimmen und insbesondere für die Netzhaut und den Kapselsack eine Sperrzone für die Laserbearbeitung zu bestimmen und dass die Steuer- und Bedieneinheit weiter ausgebildet ist, zumindest für die Sperrzonen von Netzhaut und Kapselsack und den Laserfokus mindestens je eine Markierung zu generieren, deren Charakteristik der jeweiligen Tiefenlage im Auge entspricht, um diese Markierungen auf der Anzeigeeinheit zur Anzeige zu bringen und mit dem Livebild zu überlagern.

Die Ablenkeinheit kann dabei über bekannte elektromechanische Ablenkspiegel (beispielsweise Galvo- oder MEMS-scanner) oder aber auch per einfacher manuell bedienter Ablenkung und/oder Verschiebung des Laserstrahls beispielsweise mittels eines handgehaltenen Kontaktglases und einer verschieb- und schwenkbaren Laserspaltlampe realisiert werden.

Erfindungsgemäß ist die Steuer- und Bedieneinheit ausgebildet, Floater und Strukturen des Auges zu lokalisieren und insbesondere für die Netzhaut und den Kapselsack eine Sperrzone für die Laserbearbeitung zu bestimmen. Weiterhin ist die Steuer- und Bedieneinheit ausgebildet zumindest für die Sperrzonen von Netzhaut und Kapselsack und den Laserspot eine Markierung zu generieren, deren Größe der jeweiligen Tiefeninformation im Auge entspricht, um diese Markierungen auf der Anzeigeeinheit zur Anzeige zu bringen und mit dem Livebild und/oder dem Scan zu überlagern.

Erfindungsgemäß wird die Aufgabe durch die Merkmale der unabhängigen Ansprüche gelöst. Bevorzugte Weiterbildungen und Ausgestaltungen sind Gegenstand der abhängigen Ansprüche.

Eine erste vorteilhafte Ausgestaltung betrifft das Messsystem zur Gewinnung von Tiefeninformationen des Auges, welches ein OCDR- oder OCT-System ist.

Eine zweite Gruppe vorteilhafter Ausgestaltungen betrifft die Eye-Tracker-Einheit, der als Bezug anteriore Augenbereiche, vorzugsweise Iris, Kapselsack, Linse bzw. Vorder- oder Rückfläche der Hornhaut, ein Kontaktglas oder eine im Glaskörper durch Laserbearbeitung gesetzte Bezugsmarkierung dient.

Eine dritte Gruppe vorteilhafter Ausgestaltungen betrifft die Anzeigeeinheit, die ausgebildet ist, weitere Abbildungen, insbesondere den Scan mit den Tiefeninformationen des Auges und/oder Übersicht über aktuelle Einstellungen und/oder Bedienelemente darzustellen. Besonders vorteilhaft ist die Anzeigeeinheit hierbei ein Touchscreen.

Weitere vorteilhafte Ausgestaltungen beziehen sich auf die Steuer- und Bedieneinheit, die weiterhin ausgebildet ist, Netzhautlandmarken, insbesondere Fovea, Makula, Sehnervenkopf oder auch Blutgefäße zu lokalisieren und eine Markierung zu generieren, um diese für die Laserbehandlung auf der Anzeigeeinheit zusätzlich zur Anzeige zu bringen

Die von der Steuer- und Bedieneinheit generierten Markierungen sind dabei hinsichtlich Farbe und/oder Struktur unterschiedlich. Besonders bevorzugt ändert die für den Laserspot generierte Markierung ihre Farbe und/oder Struktur, wenn sich der Laserspot einer der Sperrzonen oder Netzhautlandmarken nähert oder in diese eintritt.

Die Steuer- und Bedieneinheit ist weiter ausgebildet das Lasersystem abzuschalten, wenn sich der Laserfokus an eine der Sperrzonen oder Netzhautlandmarken annähert oder in diese eintritt. Für die beiden Sperrzonen sowie die Netzhautlandmarken können unterschiedliche Toleranzen für die Abschaltung das Lasersystem bei Annäherung oder Eintritt vergeben werden.

Einer besonders vorteilhaften Ausgestaltung entsprechend ist die Anordnung zur Laser-Vitreolyse von Glaskörpertrübungen in eine Spaltlampe integrierbar. Die vorliegende Erfindung betrifft eine Anordnung zur Laserbearbeitung von Augentrübungen. Es wird ein teilweise automatisiertes Therapiegerät (System) vorgeschlagen, bei dem 2-dimensionale Ansichten des Auges mit den Tiefeninformationen (Tiefenprofilen) kombinieren werden, um dessen Handhabung für einen Bediener bei der Lokalisierung der Floater im Zuge der Behandlung zu erleichtern.

Die Erfindung wird nachfolgend anhand von Ausführungsbeispielen näher beschrieben. Dazu zeigen
- Figur 1:: die Darstellung eines Fundusbildes mit überlagerten Markierungen,
- Figur 2:: die Darstellung eines Fundusbildes mit überlagerten Markierungen bei verschiedenen Fokuslagen des Laserspots und
- Figur3:: die Darstellung eines Scans mit Markierungen für die Sperrbereiche und den Laserspot.

Die vorgeschlagene Anordnung zur Laserbearbeitung von Augentrübungen Anordnung zur Laser-Vitreolyse von Glaskörpertrübungen besteht aus einem Messsystem zur Gewinnung von Tiefeninformationen des Auges, einem Lasersystem mit Ablenkeinheit, optischen Elementen zur Kopplung von Mess- und Lasersystem, einer Eye-Tracker-Einheit, einer Anzeigeeinheit und einer Steuer-und Bedieneinheit.

Insbesondere ist das Messsystem ausgebildet Tiefeninformationen des Auges in Form von Scans zur Verfügung zu stellen. Von der Eye-Tracker-Einheit werden axiale Augenbewegungen detektiert und kompensiert. Die Anzeigeeinheit ist ausgebildet zumindest eine 2D-Abbildung des Auges als Livebild darzustellen. Für das Livebild werden Bildwiederholraten von 5Hz, bevorzugt 10Hz und besonders bevorzugt 20Hz und Latenzzeiten <200ms, bevorzugt <100ms und besonders bevorzugt <35ms verwendet.

Erfindungsgemäß ist die Steuer- und Bedieneinheit ausgebildet die Tiefenlage von Augentrübungen relativ zur Tiefenlage des Laserfokus zu bestimmen und für die relative Lage der Augentrübungen in Bezug auf den Laserfokus Marker zu generieren und diese Markierungen auf der Anzeigeeinheit zur An-zeige zu bringen.

Weiterhin ist die Steuer- und Bedieneinheit ausgebildet in den vom Messsystem zur Verfügung gestellten Scans, Strukturen des Auges zu lokalisieren und insbesondere für die Netzhaut und den Kapselsack eine Sperrzone für die Laserbearbeitung zu bestimmen.

Insbesondere ist die Steuer- und Bedieneinheit weiter ausgebildet zumindest für die Sperrzonen von Netzhaut und Kapselsack und den Laserspot eine Markierung zu generieren, deren Größe der jeweiligen Tiefeninformation im Auge entspricht, um diese Markierungen auf der Anzeigeeinheit zur Anzeige zu bringen und mit dem Livebild zu überlagern.

Durch die Bestimmung der Lage des Laserfokus in Bezug auf die zu schonenden Augenstrukturen für diese Augenstrukturen generierten Sperrzonen, wird dem Arzt die Möglichkeit gegeben, Floater manuell zu behandeln. Die generierten Sperrzonen verhindern, dass der Laserfokus in diese eintreten und Augenstrukturen verletzten könnte.

Einer vorteilhaften Ausgestaltung entsprechend ist die Steuer- und Bedieneinheit weiterhin ausgebildet die Tiefenlage von Augentrübungen relativ zur Tiefenlage des Laserfokus zu bestimmen und für die relative Lage der Augentrübungen in Bezug auf den Laserfokus Marker zu generieren und diese Markierungen auf der Anzeigeeinheit zur Anzeige zu bringen.

Durch die zusätzliche Bestimmung der Lage von Augentrübungen (Floatern) in Bezug auf die zu schonenden Augenstrukturen bzw. auf die für diese Augenstrukturen generierten Sperrzonen, ergibt sich die Möglichkeit Floater automatisch zu behandeln.

Eine ebenfalls noch mögliche manuelle Behandlung wird dadurch vereinfacht, dass der Arzt der Lage des Laserfokus in Bezug auf die Floater zumindest näherungsweise einschätzen kann.

Als Messsystem zur Gewinnung von Tiefeninformationen des Auges wird bei der hier vorgeschlagenen Anordnung ein OCDR- oder OCT-System verwendet, das vorzugsweise mit einem YAG-Lasersystem mit Ablenkeinheit kombiniert wird.

Bei der Verwendung eines OCDR-Systems werden ein A-Scan und zusätzlich, mit Hilfe einer weiteren Bildaufnahmeeinheit, Aufnahmen des Fundus aufgenommen, wobei die Lage des OCDR-Messstrahles in Bezug auf den Fundus durch Kalibrierung bekannt ist. Bevorzugt werden hierbei die Aufnahmen des Fundus bei IR- oder NIR-Beleuchtung, zwischen 780nm und 1060nm aufgenommen.

Im Gegensatz dazu wird bei der Verwendung eines das OCT-Systems ein 3D-Volumenscan aufgenommen, wobei von der Eye-Tracker-Einheit die Augenbewegungen während der Aufnahme des 3D-Volumenscans detektiert und kompensiert werden. Eine Kompensation der Augenbewegungen ist erforderlich, da das Aufnehmen eines 3D-Volumenscans bis zu 2s dauern kann und Augenbewegungen dazu führen würden, dass die Scans deformiert wären.

Während die sich Augenstrukturen in den OCDR- oder OCT-Signalen relativ einfach detektieren lassen, sieht das bei Floatern anders aus.

Die Erkennung erfolgt vorzugsweise derart, dass zunächst das Streusignalniveau des Glaskörpers bestimmt wird. Als Floater werden solche Strukturen erkannt, die:
- um 2dB, bevorzugt 5dB höhere Signalwerte aufweisen als das mittlere Glaskörpersignal,
- die eine axiale Mindestgröße, beispielsweise >15µm in Gewebe oder einen äquivalenten optischen Weg bei angenommener Brechzahl von 1,36 aufweisen und bei denen posterior noch Signale auf Niveau des Glaskörpers auftreten oder
- deren Signalcharakteristika bekannten, in einer Datenbank gespeicherten Floatern entsprechen, wobei Größen- oder Lageverhältnisse genutzt werden.

Die Eye-Tracker-Einheit ist ausgebildet die axiale Augenbewegungen zu detektieren und zu kompensieren.

Als Bezug dienen insbesondere anteriore Augenbereiche, vorzugsweise Kapselsack, Linse bzw. Vorder- oder Rückfläche der Hornhaut dienen.

Da die Retina von Floatern abgeschatteter sein kann, wird diese in der Regel nicht als Bezug verwendet. Deshalb werden für das Tracking der axialen Augenbewegung in Bezug auf die Floater anteriorer gelegener Augenbereiche bevorzugt.

Es ist aber auch möglich als Bezug für die Eye-Tracker-Einheit das Kontaktglas oder eine im Glaskörper durch Laserbearbeitung gesetzte Bezugsmarkierung zu verwenden. Soll das Kontaktglas als Bezug verwendet werden, ist eine Referenzierung in Form einer Funktionsbeschichtung denkbar, um ein stabiles Tracking zu ermöglichen.

Erfindungsgemäß ist die Anzeigeeinheit ausgebildet neben einer 2D-Abbildung des Auges als Livebild weitere Abbildungen, insbesondere den Scan mit den Tiefeninformationen des Auges und/oder Übersicht über aktuelle Einstellungen und/oder Bedienelemente darzustellen. Dabei ist es besonders vorteilhaft, wenn die Anzeigeeinheit ein Touchscreen ist.

Als Anzeigeeinheit sind erfindungsgemäß auch Binokulare, 3D-Monitore, HMD's o. ä. vorgesehen. Weiterhin ist die Ausbildung der Steuer- und Bedieneinheit erfindungswesentlich. Insbesondere werden von ihr für die Sperrzonen von Netzhaut und Kapselsack und den Laserspot Markierung generiert, um diese auf der Anzeigeeinheit zur Anzeige zu bringen und mit dem Livebild und/oder Scan zu überlagern. Insbesondere kann hierbei auch der Floater selbst mit einer Markierung versehen werden.

Die Steuer- und Bedieneinheit ist weiter ausgebildet Netzhautlandmarken, insbesondere Fovea, Makula, Sehnervenkopf oder auch Blutgefäße zu lokalisieren und je eine Markierung für diese zu generieren, um sie gegebenenfalls auch auf der Anzeigeeinheit zur Anzeige zu bringen und mit dem Livebild und/oder Scan zu überlagern.

Um Verletzungen zu vermeiden kann die Laserbehandlung unterbrochen werden, wenn der Laserstrahl auf eine der Netzhautlandmarken gerichtet ist.

Des Weiteren ist erfindungsmäßig vorgesehen, lokale Höhenlageveränderung der Netzhaut zu überwachen, wozu kritische lokale Stellen ebenfalls, wie beschrieben markiert werden können, um bei einer weiteren Laserbehandlung Spannungen, die Netzhautablösungen verursachen könnten, zu vermeiden.

Erfindungsgemäß sind die von der Steuer- und Bedieneinheit generierten Markierungen hinsichtlich Farbe und/oder Struktur unterschiedlich.

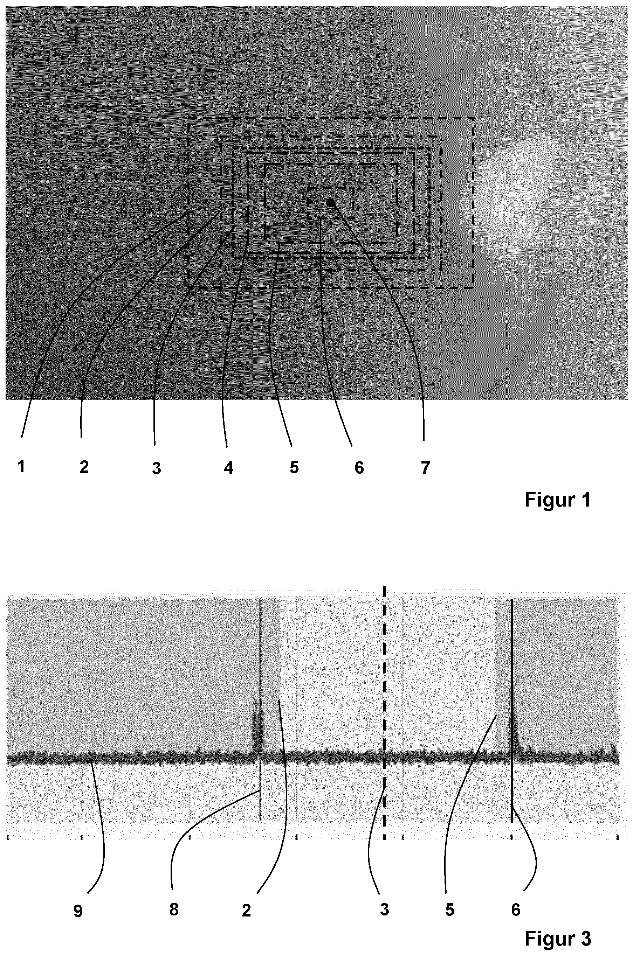

Hierzu zeigt die **Figur 1** die Darstellung eines Fundusbildes mit überlagerten Markierungen, wobei sowohl das Fundusbild als auch die Markierungen in der Realität farbig sind. Von außen nach innen bezeichnen die Markierungen: Kapselsack **1,** anteriore Sperrzonengrenze **2,** Laserfokus **3,** Floater **4,** posteriore Sperrzonengrenze **5,** Netzhaut **6** und Laserrichtung **7.**

Des Weiteren ändert die von der Steuer- und Bedieneinheit für den Laserspot generierte Markierung ihre Farbe und/oder Struktur, wenn sich der Laserspot einer der Sperrzonen oder Netzhautlandmarken nähert.

Auch ist es möglich, den Bediener bei Annäherung an oder Eintritt des Laserfokus in eine der Sperrzonen oder Netzhautlandmarken akustisch und/oder optisch zu warnen oder das Lasersystem abzuschalten.

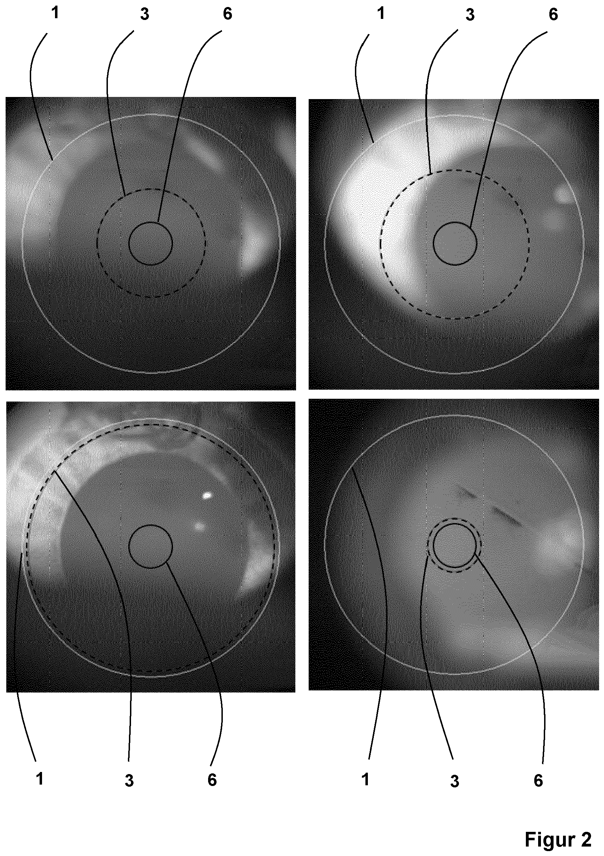

Hierzu zeigt die **Figur 2** die Darstellung eines Fundusbildes mit überlagerten Markierungen bei verschiedenen Fokuslagen des Laserspots, wobei auch hier sowohl das Fundusbild als auch die Markierungen in der Realität farbig sind. In den hier dargestellten Fundusbildern sind für eine bessere Übersichtlichkeit nur die Markierungen für die Netzhaut **6,** den Laserfokus **3** und den Kapselsack **1** vorhanden. Während in den beiden oberen Abbildungen eine Laserbehandlung möglich ist, weil sich der Laserfokus **3** in dem "sicheren" Bereich zwischen Netzhaut **6** und Kapselsack **1** befindet, ist im Gegensatz dazu in den beiden unteren Abbildungen eine Laserbehandlung nicht möglich, da sich der Laserfokus **3** zu dicht an die Netzhaut **6** bzw. den Kapselsack **1** annähert und sich jeweils in deren (nicht mit dargestellten) Sperrzone befindet.

Die **Figur 3** zeigt die Darstellung eines Scans mit Markierungen für die Sperrbereiche und den Laserspot. Aus dieser Darstellung des Tiefenscans **9** kann der Benutzer sehr schnell und sicher erkennen in welcher Tiefe sich der Laserfokus **3** in Bezug auf die Netzhaut **6** und die Linse **8** (bzw. dem Kapselsack **1)** befindet. Zusätzlich sind hier noch die posteriore Sperrzone **5** und die anteriore Sperrzone **2** dargestellt.

Weiterhin ist die Steuer- und Bedieneinheit ausgebildet für beide Sperrzonen sowie die Netzhautlandmarken unterschiedliche Toleranzen für die Annäherung zu vergeben.

In einer besonders vorteilhaften Ausgestaltung ist die Anordnung zur Laser-Vitreolyse von Glaskörpertrübungen in eine Spaltlampe integriert. Allerdings ist das hier vorgeschlagene Konzept zur Laser-Vitreolyse ebenfalls bei Operationsmikroskopen in ähnlicher Weise anwendbar.

Die vorgeschlagene Anordnung ist weiterhin geeignet, eine Art Behandlungshistorie darzustellen, beispielsweise durch Markierung und Speicherung von Schusstiefen und -positionen. Möglich ist auch die schrittweise Lageveränderung von Floatern (als Trajektorie) und auch Netzhautabschnitten beim schrittweisen Schießen mit dem Laser darzustellen (Markieren von lokalen Anhebungen).

Mit der erfindungsgemäßen Lösung wird eine Anordnung zur Laserbearbeitung von Augentrübungen zur Verfügung gestellt, die die Nachteile der bekannten technischen Lösungen behebt und eine Möglichkeit bietet 2-dimensionale Ansichten des Auges mit den 3-dimensionalen Aufnahmen des Messsystems auf einfache Art zu kombinieren und so deren Handhabung für den Bediener erleichtern. Zudem ist die Lösung leicht zu implementieren und wirtschaftlich kostengünstig und ermöglicht eine einfachere, schnellere und vor allem sicherere Behandlung störender Glaskörpertrübungen durch Laser-Vitreolyse.

Erfindungsgemäß werden die aus OCDR- oder OCT-Systemen gewonnen Tiefeninformationen bzgl. der Lage empfindlicher Augenstrukturen, dem Laserfokus und dem sich gegebenenfalls auch bewegenden, zu bearbeitenden Floater so aufbereitet, dass sie mit der dem Arzt gewohnten 2D-Ansicht in intuitiv verarbeitbarer Weise kombiniert werden können.

## Patentansprüche

1. Anordnung zur Laserbearbeitung von Augentrübungen, bestehend aus einem Messsystem zur Gewinnung von Tiefeninformationen von Augenstrukturen, einem Lasersystem zum Zerkleinern von Augentrübungen mit optischen Elementen zur Kopplung von Mess- und Lasersystem, einer Eye-Tracker-Einheit zum Detektieren axialer Augenbewegungen, einer Anzeigeeinheit und einer Steuer- und Bedieneinheit, **dadurch gekennzeichnet, dass** das Messsystem ausgebildet ist Tiefeninformationen von Augenstrukturen in Form von Tiefenprofilen zur Verfügung zu stellen, dass die Anzeigeeinheit ausgebildet ist zumindest eine 2D-Abbildung des Auges als Livebild darzustellen, dass die Steuer- und Bedieneinheit ausgebildet ist aus den Tiefenprofilen die Tiefenlage von Augenstrukturen relativ zur Tiefenlage des Laserfokus zu bestimmen und für die Netzhaut und den Kapselsack eine Sperrzone für die Laserbearbeitung zu bestimmen und dass die Steuer- und Bedieneinheit weiter ausgebildet ist, zumindest für die Sperrzonen von Netzhaut und Kapselsack und den Laserfokus mindestens je eine Markierung zu generieren, deren Charakteristik der jeweiligen Tiefenlage im Auge entspricht, um diese Markierungen auf der Anzeigeeinheit zur Anzeige zu bringen und mit dem Livebild zu überlagern.

2. Anordnung nach Anspruch 1, **dadurch gekennzeichnet, dass** die Steuer-und Bedieneinheit ausgebildet ist die Tiefenlage von Augentrübungen relativ zur Tiefenlage des Laserfokus zu bestimmen und für die relative Lage der Augentrübungen in Bezug auf den Laserfokus Marker zu generieren und diese Markierungen auf der Anzeigeeinheit zur Anzeige zu bringen.

3. Anordnung nach Anspruch 2, **dadurch gekennzeichnet, dass** die Steuer-und Bedieneinheit ausgebildet ist die Charakteristik der zu generierenden Markierung hinsichtlich Farbe, Form, numerischen Wertes der Tiefenlage, aber bevorzugt deren Größe zu variieren.

4. Anordnung nach Anspruch 1, **dadurch gekennzeichnet, dass** die Steuer-und Bedieneinheit ausgebildet ist, die Markierungen mit tiefenlagenabhängiger Größe für Netzhaut, Kapselsack, Linse, Laserfokus, Augentrübungen so zu generieren, dass die Markierungen um die laterale Laserfokuslage angeordnet sind, bevorzugt zentriert.

5. Anordnung nach Anspruch 1, **dadurch gekennzeichnet, dass** die Eye-Tracker-Einheit ausgebildet ist, axiale Augenbewegungen zu kompensieren.

6. Anordnung nach Anspruch 1, **dadurch gekennzeichnet, dass** die eine 2D-Abbildung eine en face-Abbildung des hinteren Augenabschnitts ist, die mit einer Bildwiederholrate von 5Hz, bevorzugt 10Hz und besonders bevorzugt 20Hz und einer Latenzzeit <200ms, bevorzugt <100ms und besonders bevorzugt <35ms als Livebild dargestellt wird.

7. Anordnung nach Anspruch 1, **dadurch gekennzeichnet, dass** das Messsystem zur Gewinnung von Tiefeninformationen des Auges ein OCDR-System ist.

8. Anordnung nach Anspruch 1, **dadurch gekennzeichnet, dass** als Bezug für die Eye-Tracker-Einheit anteriore Augenbereiche, vorzugsweise Kapselsack, Linse bzw. Vorder- oder Rückfläche der Hornhaut dienen.

9. Anordnung nach Anspruch 1, **dadurch gekennzeichnet, dass** als Bezug für die Eye-Tracker-Einheit ein Kontaktglas oder eine im Glaskörper durch Laserbearbeitung gesetzte Bezugsmarkierung dient.

10. Anordnung nach Anspruch 1 und 6, **dadurch gekennzeichnet, dass** das OCDR-System ausgebildet ist einen A-Scan aufzunehmen und dass zusätzlich eine Bildaufnahmeeinheit für Aufnahmen des Fundus vorhanden ist, wobei die Lage des OCDR-Messstrahles in Bezug auf den Fundus durch Kalibrierung bekannt ist.

11. Anordnung nach Anspruch 1 und 6, **dadurch gekennzeichnet, dass** das OCT-System ausgebildet ist einen 3D-Volumenscan aufzunehmen und die Eye-Tracker-Einheit ausgebildet ist detektierte Augenbewegungen während der Aufnahme des 3D-Volumenscans zu kompensieren.

12. Anordnung nach Anspruch 1, **dadurch gekennzeichnet, dass** die Anzeigeeinheit ausgebildet ist weitere Abbildungen, insbesondere den Scan mit den Tiefeninformationen des Auges und/oder Übersicht über aktuelle Einstellungen und/oder Bedienelemente darzustellen.

13. Anordnung nach Anspruch 1 und 11, **dadurch gekennzeichnet, dass** die Anzeigeeinheit ein Touchscreen ist.

14. Anordnung nach Anspruch 1, **dadurch gekennzeichnet, dass** die Steuer-und Bedieneinheit ausgebildet ist die für die Sperrzonen von Netzhaut und Kapselsack und den Laserfokus generierten Markierungen auf der Anzeigeeinheit zur Anzeige zu bringen und mit dem Scan zu überlagern.

15. Anordnung nach Anspruch 1, **dadurch gekennzeichnet, dass** die Steuer-und Bedieneinheit ausgebildet ist Netzhautlandmarken, insbesondere Fovea, Makula, Sehnervenkopf oder auch Blutgefäße zu lokalisieren und eine Markierung für die Netzhautlandmarken zu generieren.

16. Anordnung nach Anspruch 2, **dadurch gekennzeichnet, dass** die von der Steuer- und Bedieneinheit generierten Markierungen hinsichtlich Farbe und/oder Struktur unterschiedlich sind.

17. Anordnung nach Anspruch 1, **dadurch gekennzeichnet, dass** die von der Steuer- und Bedieneinheit für den Laserspot generierte Markierung ihre Farbe und/oder Struktur ändert, wenn sich der Laserspot einer der Sperrzonen oder Netzhautlandmarken nähert.

18. Anordnung nach Anspruch 1, **dadurch gekennzeichnet, dass** die Steuer-und Bedieneinheit ausgebildet den Bediener bei Annäherung des Lasersystems an eine der Sperrzonen oder Netzhautlandmarken akustisch und/oder optisch zu warnen.

19. Anordnung nach Anspruch 1, **dadurch gekennzeichnet, dass** die Steuer-und Bedieneinheit ausgebildet ist das Lasersystem bei Annäherung an oder Eintritt in eine der Sperrzonen oder Netzhautlandmarken abzuschalten.

20. Anordnung nach Anspruch 1, **dadurch gekennzeichnet, dass** die Steuer-und Bedieneinheit ausgebildet ist für beide Sperrzonen sowie die Netzhautlandmarken unterschiedliche Toleranzen für die Abschaltung das Lasersystem bei Annäherung vergeben kann.

21. Anordnung nach Anspruch 1, **dadurch gekennzeichnet, dass** die Anordnung zur Laser-Vitreolyse von Glaskörpertrübungen in eine Spaltlampe integrierbar ist.

## Claims

1. Arrangement for laser treatment of eye opacification, consisting of
a measuring system for obtaining depth information relating to eye structures, a laser system for comminuting eye opacification and having optical elements for coupling the measuring and laser system, an eye tracker unit for detecting axial eye movements, a display unit, and a control and operating unit, **characterized in that** the measuring system is designed to make available depth information relating to eye structures in the form of depth profiles, **in that** the display unit is designed to display at least one 2-D image representation of the eye as a live image, **in that** the control and operating unit is designed to determine the depth of eye structures relative to the depth of the laser focus from the depth profiles and to determine an exclusion zone for laser treatment, for the retina and the capsular bag, and **in that** the control and operating unit is further designed to generate at least one marking for at least each of the exclusion zones of the retina and capsular bag and the laser focus, the characteristics of which markings correspond to the respective depth in the eye, in order to display these markings on the display unit and overlay these on the live image.

2. Arrangement according to Claim 1, **characterized in that** the control and operating unit is designed to determine the depth of eye opacification relative to the depth of the laser focus and to generate markers for the relative position of eye opacification in relation to the laser focus and to display these markings on the display unit.

3. Arrangement according to Claim 2, **characterized in that** the control and operating unit is designed to vary the characteristics of the marking to be generated with regard to colour, shape, numerical value of the depth, but preferably with regard to its size.

4. Arrangement according to Claim 1, **characterized in that** the control and operating unit is designed to generate the markings with depth-dependent size for retina, capsular bag, lens, laser focus, eye opacification in such a way that the markings are arranged, preferably centred, around the lateral laser focus position.

5. Arrangement according to Claim 1, **characterized in that** the eye tracker unit is designed to compensate for axial eye movements.

6. Arrangement according to Claim 1, **characterized in that** the one 2-D image representation is an en face image representation of the posterior segment of the eye which is displayed as a live image with a refresh rate of 5 Hz, preferably 10 Hz, and particularly preferably 20 Hz, and a latency <200 ms, preferably <100 ms and particularly preferably <35 ms.

7. Arrangement according to Claim 1, **characterized in that** the measuring system for obtaining depth information of the eye is an OCDR system.

8. Arrangement according to Claim 1, **characterized in that** anterior regions of the eye, preferably the capsular bag, lens or front or back surface of the cornea, serve as a reference for the eye tracker unit.

9. Arrangement according to Claim 1, **characterized in that** a contact glass or a reference marking placed in the vitreous humour by laser treatment serves as a reference for the eye tracker unit.

10. Arrangement according to Claims 1 and 6, **characterized in that** the OCDR system is designed to record an A-scan and **in that** there is also an image recording unit for making recordings of the fundus, with the position of the OCDR measuring beam in relation to the fundus being known through calibration.

11. Arrangement according to Claims 1 and 6, **characterized in that** the OCT system is designed to record a 3-D volume scan and the eye tracker unit is designed to compensate for eye movements detected during the recording of the 3-D volume scan.

12. Arrangement according to Claim 1, **characterized in that** the display unit is designed to display further image representations, in particular the scan with the depth information of the eye and/or an overview of current settings and/or operating elements.

13. Arrangement according to Claims 1 and 11, **characterized in that** the display unit is a touch screen.

14. Arrangement according to Claim 1, **characterized in that** the control and operating unit is designed to display on the display unit the markings generated for the exclusion zones of the retina and capsular bag and for the laser focus and to overlay these on the scan.

15. Arrangement according to Claim 1, **characterized in that** the control and operating unit is designed to localize retinal landmarks, in particular the fovea, macula, optic nerve head or blood vessels, and to generate a marking for the retinal landmarks.

16. Arrangement according to Claim 2, **characterized in that** the markings generated by the control and operating unit differ in terms of colour and/or structure.

17. Arrangement according to Claim 1, **characterized in that** the marking generated by the control and operating unit for the laser spot changes its colour and/or structure when the laser spot approaches one of the exclusion zones or retinal landmarks.

18. Arrangement according to Claim 1, **characterized in that** the control and operating unit is designed to warn the operator acoustically and/or optically when the laser system approaches one of the exclusion zones or retinal landmarks.

19. Arrangement according to Claim 1, **characterized in that** the control and operating unit is designed to switch off the laser system should the latter approach or enter one of the exclusion zones or retinal landmarks.

20. Arrangement according to Claim 1, **characterized in that** the control and operating unit is designed to be able to assign different tolerances for switching off the laser system when approaching the two exclusion zones and the retinal landmarks.

21. Arrangement according to claim 1, **characterized in that** the arrangement for laser vitreolysis of vitreous humour opacification is able to be integrated into a slit lamp.

## Revendications

1. Ensemble de traitement laser d'opacités oculaires, ledit ensemble comprenant un système de mesure destiné à obtenir des informations de profondeur de structures oculaires, un système laser destiné à fractionner les opacités oculaires et comprenant des éléments optiques destinés à coupler le système de mesure et le système laser, une unité de suivi oculaire destinée à détecter les mouvements oculaires axiaux, une unité d'affichage et une unité de commande et d'utilisation, **caractérisé en ce que** le système de mesure est conçu pour fournir des informations de profondeur de structures oculaires sous forme de profils de profondeur, **en ce que** l'unité d'affichage est conçue pour représenter au moins une reproduction 2D de l'œil comme une image en direct, **en ce que** l'unité de commande et d'utilisation est conçue pour déterminer à partir des profils de profondeur la position en profondeur de structures oculaires par rapport à la position en profondeur du foyer laser et pour déterminer une zone d'exclusion du traitement laser pour la rétine et le sac capsulaire et **en ce que** l'unité de commande et d'utilisation est en outre conçue pour générer, au moins pour les zones d'exclusion de la rétine et du sac capsulaire et pour le foyer laser, à chaque fois au moins un marquage dont la caractéristique correspond à la position en profondeur respective dans l'œil, afin d'afficher ces marquages sur l'unité d'affichage et de les superposer à l'image en direct.

2. Ensemble selon la revendication 1, **caractérisé en ce que** l'unité de commande et d'utilisation est conçue pour déterminer la position en profondeur des opacités oculaires par rapport à la position en profondeur du foyer laser et générer des marqueurs pour la position relative des opacités oculaires par rapport au laser et afficher ces marquages sur l'unité d'affichage.

3. Ensemble selon la revendication 2, **caractérisé en ce que** l'unité de commande et d'utilisation est conçue pour faire varier la caractéristique du marquage à générer en termes de couleur, de forme, de valeur numérique de la position en profondeur, mais de préférence de taille de celle-ci.

4. Ensemble selon la revendication 1, **caractérisé en ce que** l'unité de commande et d'utilisation est conçue pour générer les marquages avec une taille dépendant de la position en profondeur pour la rétine, le sac capsulaire, le cristallin, le foyer laser, les opacités oculaires de telle sorte que les marquages soient disposés autour de la position de focalisation laser latérale, de préférence de manière centrée.

5. Ensemble selon la revendication 1, **caractérisé en ce que** l'unité de suivi oculaire est conçue pour compenser les mouvements oculaires axiaux.

6. Ensemble selon la revendication 1, **caractérisé en ce que** la reproduction 2D est une reproduction de face de la portion oculaire arrière, qui est représentée avec un taux de répétition d'image de 5 Hz, de préférence de 10 Hz et de manière particulièrement préférée de 20 Hz, et un temps de latence < 200 ms, de préférence < 100 ms et de manière particulièrement préférée < 35 ms sous forme d'image en direct.

7. Ensemble selon la revendication 1, **caractérisé en ce que** le système de mesure destiné à obtenir des informations de profondeur de l'œil est un système OCDR.

8. Ensemble selon la revendication 1, **caractérisé en ce que** des zones oculaires antérieures, de préférence le sac capsulaire, le cristallin ou la surface avant ou arrière de la cornée servent de référence à l'unité de suivi oculaire.

9. Ensemble selon la revendication 1, **caractérisé en ce qu'**un verre de contact ou un marquage de référence placé dans le corps vitré par traitement laser sert de référence à l'unité de suivi oculaire.

10. Ensemble selon les revendications 1 et 6, **caractérisé en ce que** le système OCDR est conçu pour enregistrer un A-scan et **en ce qu'**une unité d'enregistrement d'images est également présente pour enregistrer le fond d'œil, la position du faisceau de mesure OCDR par rapport au fond d'œil étant connue par étalonnage.

11. Ensemble selon les revendications 1 et 6, **caractérisé en ce que** le système OCT est conçu pour enregistrer un balayage volumique 3D et l'unité de suivi oculaire est conçue pour compenser les mouvements oculaires détectés pendant l'enregistrement du balayage volumique 3D.

12. Ensemble selon la revendication 1, **caractérisé en ce que** l'unité d'affichage est conçue pour représenter d'autres reproductions, en particulier le balayage avec les informations de profondeur de l'œil et/ou un aperçu des réglages et/ou éléments d'utilisation actuels.

13. Ensemble selon les revendications 1 et 11, **caractérisé en ce que** l'unité d'affichage est un écran tactile.

14. Ensemble selon la revendication 1, **caractérisé en ce que** l'unité de commande et d'utilisation est conçue pour afficher les marquages générés pour les zones d'exclusion de la rétine et du sac capsulaire et le foyer laser sur l'unité d'affichage et pour les superposer au balayage.

15. Ensemble selon la revendication 1, **caractérisé en ce que** l'unité de commande et d'utilisation est conçue pour localiser des repères rétiniens, notamment la fovéa, la macula, la tête du nerf optique ou les vaisseaux sanguins et pour générer un marquage pour les repères rétiniens.

16. Ensemble selon la revendication 2, **caractérisé en ce que** les marquages générés par l'unité de commande et d'utilisation sont différents en termes de couleur et/ou de structure.

17. Ensemble selon la revendication 1, **caractérisé en ce que** le marquage généré par l'unité de commande et d'utilisation du spot laser change de couleur et/ou de structure lorsque le spot laser se rapproche d'une des zones d'exclusion ou d'un des repères rétiniens.

18. Ensemble selon la revendication 1, **caractérisé en ce que** l'unité de commande et d'utilisation est conçue pour avertir l'opérateur par des moyens acoustiques et/ou optiques lorsque le système laser s'approche d'une des zones d'exclusion ou d'un des repères rétiniens.

19. Ensemble selon la revendication 1, **caractérisé en ce que** l'unité de commande et d'utilisation est conçue pour désactiver le système laser à l'approche d'une des zones d'exclusion ou d'un des repères rétiniens ou à l'entrée dans celle-ci/celui-ci.

20. Ensemble selon la revendication 1, **caractérisé en ce que** l'unité de commande et d'utilisation est conçue aussi bien pour les zones d'exclusion que pour les repères rétiniens et peut attribuer différentes tolérances pour la désactivation du système laser lors de l'approche.

21. Ensemble selon la revendication 1, **caractérisé en ce que** l'ensemble de vitréolyse laser des opacités de corps vitré peut être intégré dans une lampe à fente.
